(19) **Europäisches Patentamt European Patent Office Office européen des brevets**

(11) **EP 4 153 047 B1**

(12) **EUROPEAN PATENT SPECIFICATION**

(45) Date of publication and mention
of the grant of the patent:
**29.01.2025 Bulletin 2025/05**

(21) Application number: **21732996.0**

(22) Date of filing: **21.05.2021**

(51) International Patent Classification (IPC):
*A61B 5/11* (2006.01)    *G01S 7/40* (2006.01)
*G01S 7/41* (2006.01)    *G01S 13/42* (2006.01)
*G01S 13/58* (2006.01)    *G01S 13/86* (2006.01)
*G01S 13/88* (2006.01)    *G08B 21/04* (2006.01)
*G08B 25/01* (2006.01)    *G01S 7/48* (2006.01)
*G01S 7/497* (2006.01)    *G01S 17/46* (2006.01)
*G01S 17/86* (2020.01)    *G01S 17/88* (2006.01)
*G01S 17/42* (2006.01)    *G01S 13/34* (2006.01)
*G01S 15/88* (2006.01)    *G08B 13/181* (2006.01)

(52) Cooperative Patent Classification (CPC):
**G01S 17/46; A61B 5/1117; G01S 7/4034;
G01S 7/411; G01S 7/415; G01S 7/4802;
G01S 7/4972; G01S 13/42; G01S 13/584;
G01S 13/86; G01S 13/886; G01S 17/42;
G01S 17/86; G01S 17/88; G08B 21/043;** (Cont.)

(86) International application number:
**PCT/IL2021/050593**

(87) International publication number:
**WO 2021/234714 (25.11.2021 Gazette 2021/47)**

(54) **A DEVICE FOR MEASURING 3D-COORDINATES ASSOCIATED WITH AN OBJECT**

VORRICHTUNG ZUR MESSUNG VON 3D-KOORDINATEN IN VERBINDUNG MIT EINEM OBJEKT

DISPOSITIF DE MESURE DE COORDONNÉES 3D ASSOCIÉES À UN OBJET

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.05.2020 GB 202008033**

(43) Date of publication of application:
**29.03.2023 Bulletin 2023/13**

(73) Proprietor: **Essence Smartcare Ltd.
4672530 Herzlia Pituach (IL)**

(72) Inventors:
• **KATZ, Barak**
4906217 Petach Tikva (IL)
• **AMIR, Ohad**
4642300 Herzlia (IL)

(74) Representative: **Marks & Clerk LLP
15 Fetter Lane
London EC4A 1BW (GB)**

(56) References cited:
US-A1- 2016 040 982    US-A1- 2016 077 202
US-A1- 2019 195 728    US-B1- 7 916 066

(52) Cooperative Patent Classification (CPC): (Cont.)
**G08B 21/0438;** G01S 13/34; G01S 15/88;
G08B 13/181

**Description**

TECHNICAL FIELD

The present invention relates generally to device for measuring 3D coordinates associated with an object in an environment, and a method of using such a device. In particular, embodiments of the invention relate to alignment of the device in an environment.

BACKGROUND

A 3D reflected wave measuring device can be used to identify the height of locations from which waves are reflected to the measuring device (and detected as reflected wave measurements). This may be achieved by subtracting a vertical coordinate representing a surface (with respect to which the height is to be referenced) from a vertical coordinate that corresponds to the reflection location.

However, for a 3D reflected wave measuring device having a three dimensional coordinate system the reflected wave measuring device may be installed in an orientation that is not aligned with an expected a frame of reference within the environment, which may result in incorrect determinations of heights.

US 2019/195728 A1 discloses a radar-based structural monitoring system configured to detect structural damage of buildings or catastrophic failure, such as an object having fallen over. The system comprises a radar sensor configured to monitor a target in two or three dimensions and a three-axis accelerometer that is physically attached the radar sensor and monitors the motion of the radar sensor to compensate for a self-motion of the radar sensor present in the measured radar data.

US 2016/077202 A1 discloses a navigational aid comprising an adaptively beam-forming radar sensor. The sensor detects its own orientation, notifies a user of a the need for re-alignment if necessary, and compensates a detected tilt of the device by steering the beam substantially to the opposite direction by means of motors.

US 2016/040982 A1 discloses a 3D measurement system comprising a range camera and an orientation sensor. During an alignment process, a user is guided with messages.

US 7 916 066 B1 discloses a radar-based ceiling-mounted fall sensor configured to detect if a person falls based on obtained 3D data.

SUMMARY OF THE INVENTION

The inventors have identified that 3D reflected wave measuring devices have applications in the field of fall detectors or security. As such, determining a correct height metric associated with an object of interest within an environment may be important.

Therefore, the present invention provides a device according to claim 1.

The orientation sensor advantageously enables the 3D reflected wave measuring device to be aligned correctly to a frame of reference within an environment. This in turn enables a more accurate measurement of 3D reflected wave data points from an object within the environment to be made.

The present invention also provides a method according to claim 8. Further advantageous embodiments are provided by the dependent claims.

Any instructions may be provided on one or more carriers. For example there may be one or more non-transient memories, e.g. a EEPROM (e.g. a flash memory) a disk, CD- or DVD-ROM, programmed memory such as read-only memory (e.g. for Firmware), one or more transient memories (e.g. RAM), and/or a data carrier(s) such as an optical or electrical signal carrier. The memory/memories may be integrated into a corresponding processing chip and/or separate to the chip. Code (and/or data) to implement embodiments of the present disclosure may comprise source, object or executable code in a conventional programming language (interpreted or compiled) such as C, or assembly code, code for setting up or controlling an ASIC (Application Specific Integrated Circuit) or FPGA (Field Programmable Gate Array), or code for a hardware description language.

These and other aspects will be apparent from the embodiments described in the following. The scope of the present disclosure is not intended to be limited by this summary nor to implementations that necessarily solve any or all of the disadvantages noted.

BRIEF DESCRIPTION OF THE SEVERAL VIEWS OF THE DRAWINGS

The present invention will now be described, by way of example only, and with reference to the accompanying drawings, in which:

> Figure 1 illustrates an environment in which a device has been positioned;
> Figure 2 is a schematic block diagram of the device;
> Figures 3a and 3b illustrates a human body with indications of reflections measured by a reflective wave detector when the person is in a standing non-fall state and in a fall state; and
> Figures 4a and 4b illustrate the issue with misaligned frames of reference in a height metric determination.

DETAILED DESCRIPTION

In the following detailed description, reference is made to the accompanying drawings that form a part hereof, and in which is shown by way of illustration specific embodiments of the invention. These embodiments are described in sufficient detail to enable those skilled in the art to practice them, and it is to be understood that other embodiments may be utilized, and that structural, logical, and electrical changes may be made without departing from the scope of the invention.

The following description is, therefore, not to be taken in a limited sense, and the scope of the invention is defined by the appended claims. In the following embodiments, like components are labelled with like reference numerals.

In the following embodiments, the term data store or memory is intended to encompass any computer readable storage medium and/or device (or collection of data storage mediums and/or devices). Examples of data stores include, but are not limited to, optical disks (e.g., CD-ROM, DVD-ROM, etc.), magnetic disks (e.g., hard disks, floppy disks, etc.), memory circuits (e.g., EEPROM, solid state drives, random-access memory (RAM), etc.), and/or the like.

As used herein, except wherein the context requires otherwise, the terms "comprises", "includes", "has" and grammatical variants of these terms, are not intended to be exhaustive. They are intended to allow for the possibility of further additives, components, integers or steps.

The functions or algorithms described herein are implemented in hardware, software or a combination of software and hardware in one or more embodiments. The software comprises computer executable instructions stored on computer readable carrier media such as memory or other type of storage devices. Further, described functions may correspond to modules, which may be software, hardware, firmware, or any combination thereof. Multiple functions are performed in one or more modules as desired, and the embodiments described are merely examples. The software is executed on a digital signal processor, ASIC, microprocessor, or other type of processor.

Specific embodiments will now be described with reference to the drawings.

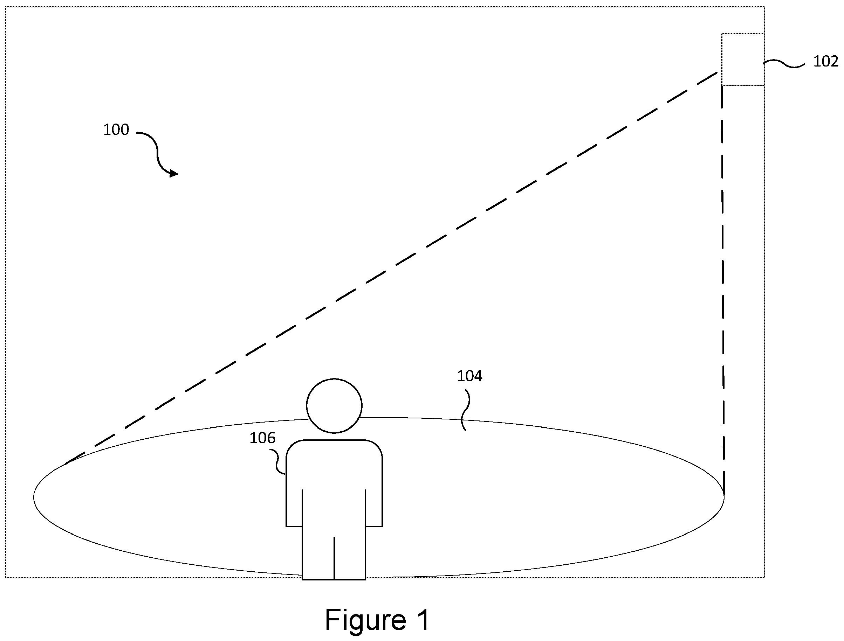

Figure 1 illustrates an environment 100 in which a device 102 has been positioned. The environment 100 may for example be an indoor space such as a room of a home, a nursing home, a public building or other indoor space. Alternatively the environment may be an outdoor space such as a garden. The device 102 is configured to monitor a space 104 in the environment 100 in which a person 106 may be present.

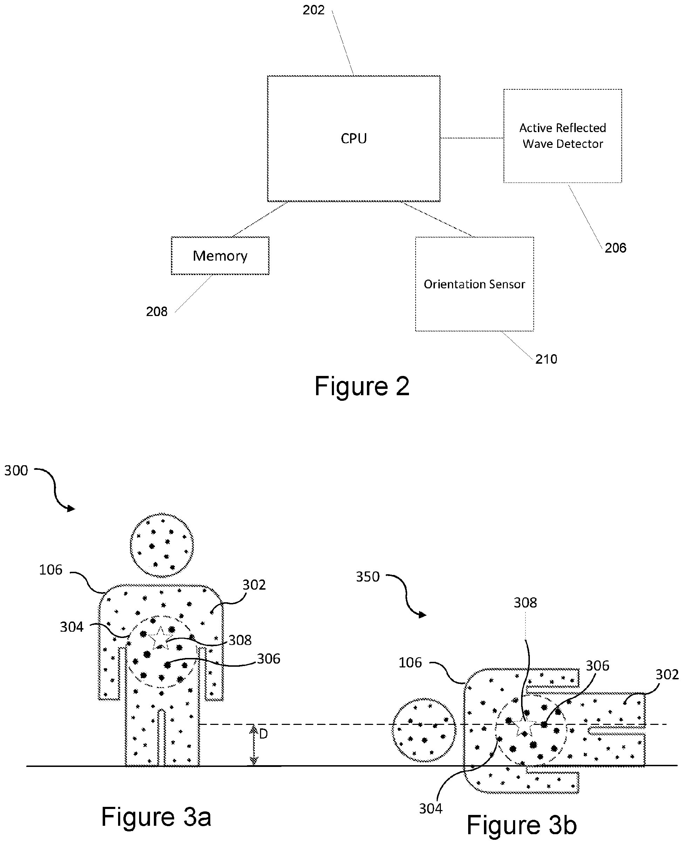

Figure 2 illustrates a simplified view of the device 102. A shown in Figure 2, the device 102 comprises a central processing unit ("CPU") 202, to which is connected a memory 208. The functionality of the CPU 202 described herein may be implemented in code (software) stored on a memory (e.g. memory 208) comprising one or more storage media, and arranged for execution on a processor comprising on or more processing units. The storage media may be integrated into and/or separate from the CPU 202. The code is configured so as when fetched from the memory and executed on the processor to perform operations in line with embodiments discussed herein. Alternatively it is not excluded that some or all of the functionality of the CPU 202 is implemented in dedicated hardware circuitry, or configurable hardware circuitry like an FPGA.

Figure 2 shows the CPU 202 being connected to an active reflected wave detector 206 (also referred herein as a 3D reflective wave measuring device) and an orientation sensing device 210. While in the illustrated embodiment the orientation sensing device 210 and reflected wave detector are separate from the CPU 202, in other embodiments, at least part of processing aspects of the active reflected wave detector 206 may be provided by a processor that also provides the CPU 202, and resources of the processor may be shared to provide the functions of the CPU 202 and the processing aspects active reflected wave detector 206. Similarly, functions of the CPU 202, such as those described herein, may be performed in the active reflected wave detector 206. The function and purpose of the orientation sensing device 210 will be described later.

As shown in Figure 2, the device 102 may have a housing that houses the CPU 202, active reflected wave detector 206, orientation sensor 210 and memory 208. Alternatively, the active reflected wave detector 206 may be external to the device 102 and be coupled to the CPU 202 by way of a wired or wireless connection. Further, the outputs of the active reflected wave detector 206 may be wirelessly received from via an intermediary device that relays, manipulates and/or in part produces their outputs, for example a control hub of a monitoring and/or home automation system, which may in some cases comprise a security system.

In some embodiments, the CPU 202 is configured to detect the presence of a person in the monitored space 104, and if a person is present, classify the state or postural position (standing, sitting or having fallen) of the person based on an output of the active reflected wave detector 206.

The active reflected wave detector 206 may operate in accordance with one of various reflected wave technologies.

Preferably, the active reflected wave detector 206 is a radar sensor. The radar sensor 206 may use millimeter wave (mmWave) sensing technology. The radar is, in some embodiments, a continuous-wave radar, such as frequency modulated continuous wave (FMCW) technology. Such a chip with such technology may be, for example, Texas Instruments Inc. part number IWR6843. The radar may operate in microwave frequencies, e.g. in some embodiments a carrier wave in the range of 1-100GHz (76-81Ghz or 57-64GHz in some embodiments), and/or radio waves in the 300MHz to 300GHz range, and/or millimeter waves in the 30GHz to 300GHz range. In some embodiments, the radar has a bandwidth of at least 1 GHz. The active reflected wave detector 206 may comprise antennas for both emitting waves and for receiving reflections of the emitted waves, and in some embodiment different antennas may be used for the emitting compared with the receiving.

The active reflected wave detector 206 is not limited to being a radar sensor, and in other embodiments, the active reflected wave detector 206 is a lidar sensor, or a sonar sensor.

The active reflected wave detector 206 being a radar sensor is advantageous over other reflected wave technologies in that radar signals can transmit through some materials, e.g. wood or plastic, but not others - notably water which is important because humans are mostly water. This means that the radar can potentially "see" a person in the environment 100 even if they are behind such an object. This is not the case for sonar.

In embodiments, the active reflected wave detector 206 is used to determine whether the person is in a posture that may be relate to them standing, sitting, or having fallen. This may be achieved for example by detecting a height associated with a certain location on their body, e.g. a location above their legs.

In operation, the active reflected wave detector 206 performs one or more reflected wave measurements at a given moment of time, and over time these reflected wave measurements can be correlated by the CPU 202 with the presence of a person and/or a state of the person and/or a condition of the person. In the context of the present disclosure, the state of the person may be a characterization of the person based on a momentary assessment. For example, a classification passed on their position (e.g. in a location in respect to the floor and in a configuration which are consistent or inconsistent with having fallen) and/or their kinematics (e.g. whether they have a velocity that is consistent or inconsistent with them having fallen, or having fallen possibly being immobile). In the context of the present disclosure, the condition of the person comprises a, determination of an aspect of the person's health or physical predicament, the aspect being whether they are in a fall condition whereby they have fallen and are substantially immobile, such that they may not be able (physically and/or emotionally) to get to a phone to call for help. In some embodiments this involves an assessment of the person's status over time, such as in the order or 30-60 seconds. However, the condition of the person may in some contexts be synonymous with the status of the person. For example, by determining that the person is in a safe supported state or a standing state, it may be concluded that the person is not currently in a fall condition, whereby they are on the floor and potentially unable to seek help. It may additionally or alternatively be concluded that they are in a resting condition because of their status being determined to be in a safe supported state, e.g. lying on a bed. In another example their condition may be classified as active and/or mobile based on a determination of a walking status.

Figure 3a illustrates a free-standing human body 106 with indications of reflective wave reflections therefrom in accordance with embodiments.

For each reflected wave measurement, for a specific time in a series of time-spaced reflective wave measurements, the reflective wave measurement may include a set of one or more measurement points that make up a "point cloud". Each point 302 in the point cloud may be defined by a 3-dimensional spatial position from which a reflection was received, and defining a peak reflection value, and a doppler value from that spatial position. Thus, a measurement received from a reflective object may be defined by a single point, or a cluster of points from different positions on the object, depending on its size. In some embodiments the point cloud is prefiltered to exclude static points, for example by using a moving target indication as is known in the art.

Figure 3a illustrates a map of reflections. The size of the point represents the intensity (magnitude) of energy level of the radar reflections (see larger point 306). Different parts or portions of the body reflect the emitted signal (e.g. radar) differently. For example, generally, reflections from areas of the torso 304 are stronger than reflections from the limbs. Each point represents coordinates within a bounding shape for each portion of the body. Each portion can be separately considered and have separate boundaries, e.g. the torso and the head may be designated as different portions. The point cloud can be used as the basis for a calculation of a reference parameter or set of parameters which can be stored instead of or in conjunction with the point cloud data for a reference object (human) for comparison with a parameter or set of parameters derived or calculated from a point cloud for radar detections from an object (human).

When a cluster of measurement points are received from an object in the environment 100, a location of a particular part/point on the object or a portion of the object, e.g. its centre, may be determined by the CPU 202 from the cluster of measurement point positions having regard to the intensity or magnitude of the reflections (e.g. a centre location comprising an average of the locations of the reflections weighted by their intensity or magnitude). As illustrated in figure 3a, the reference body has a point cloud from which its centre has been calculated and represented by the location 308, represented by the star shape. In this embodiment, the torso 304 of the body is separately identified from the body and the centre of that portion of the body is indicated. In alternative embodiments, the body can be treated as a whole or a centre can be determined for each of more than one body part e.g. the torso and the head, for separate comparisons with centres of corresponding portions of a scanned body.

The object's centre or portion's centre may be a weighted centre of the measurement points. The locations may be weighted according to an Radar Cross Section (RCS) estimate of each measurement point, where for each measurement point the RCS estimate may be calculated as a constant (which may be determined empirically for the reflected wave detector 206) multiplied by the signal to noise ratio for the measurement divided by $R^4$, where R is the distance from the reflected wave detector 206 antenna configuration to the position corresponding to the measurement point. In other embodiments, the RCS may be calculated as a constant multiplied by the signal for the measurement divided by $R^4$. This may be the case, for example, if the noise is constant or may be treated as though it were constant. Regardless, the received radar reflections in the exemplary embodiments described herein may be considered as an intensity value, such as an absolute value of the amplitude of a received radar signal.

In any case, the weighted centre, WC, of the measurement points for an object may be calculated for each dimension as:

$$WC = \frac{1}{\sum_{n=1}^{N} W_n} \sum_{n=1}^{N} (W_n P_n)$$

Where:

> $N$ is the number of measurement points for the object;
> $W_n$ is the RCS estimate for the $n^{th}$ measurement point; and
> $P_n$ is the location (e.g. its coordinate) for the $n^{th}$ measurement point in that dimension.

The CPU 202 may determine a height metric associated with at least one measurement of a reflection from the person conveyed in the output of the active reflected wave detector 206 and compare the height metric to at least one threshold.

The height metric may be a height of a weighted centre of the measurement points of a body or part thereof (where each measurement is weighted by the RCS estimation), and the CPU 202 may compare this height metric to a threshold distance, D, from the floor (e.g. 30cm) and determine that the person in the environment is in a standing (non-fall) position if the height metric exceeds the threshold distance, this is illustrated in Figure 3a.

The height metric is not limited to being a height of a weighted centre of the measurement points of the person's body or part thereof. In another example, the height metric may be a maximum height of all of the height measurements associated with the person's body or part thereof. In another example, the height metric may be an average height (e.g. median z value) of all of the height measurements of the person's body or part thereof. In the case of using a weighted centre or average height, the "part thereof" may beneficially be a part of the body that is above the person's legs to more confidently distinguish between fall and non-fall positions.

If the height metric (e.g. weighted centre, average height and/or maximum height) is within (less than) the threshold distance, D, from the floor (e.g. 30cm), the CPU 202 may determine that the person in the environment is in a fall position, this is illustrated in Figure 3b. If the height metric is greater than a first threshold distance from the floor but less than a second threshold distance from the floor (for example the a maximum height amongst the measurements associated with body is between 30cm and 1.3m, the CPU 202 may be able to detect that the person is in a safe reclined position, e.g. lying down on a bed or couch, which is an example of a non-fall position.

When the device is a fall detector, the detector could be a part of the device or a module of the code or part of its functionality.

**Alignment of the Active Reflective Wave Detector**

As can be seen from the above methods, a reliable and accurate determination of the value for a height metric from the Active Reflective Wave Detector is important if a reliable determination of a position or posture of the person is to be made based on data received from the active reflected wave detector. That position may, for example, be a related to postural information indicative of a person standing position, sitting position or having fallen over. As mentioned above, such data may be in the form of sets of points defined by 3D coordinates and intensities or magnitudes of the reflective wave for each of the points.

The device may or may not be installed in an orientation and position such that its active reflected wave detector is not aligned with an expected frame of reference (for example a horizontal plane lying a height $H_1$ above a point on a surface such as the floor, which in some embodiments is a horizontal surface). Thus, the expected frame of reference may have an axis $x_2, y_2, z_2$, which is associated with an environment. In particular, $x_2$ and $y_2$ are horizontal and $z_2$ is vertical, where the $x_2$-$y_2$ plane lies a distance H above the floor. A horizontal floor may thus lie in a plane defined by the equation $z_2 = -H$. The device's active reflected wave detector has an actual frame of reference (i.e. how it is actually installed) having an axis $x_1$, $y_1$ and $z_1$, in which the z axis is only nominally (but not necessarily in practice) vertical. If the nominal axis ($x_2$, $y_2$, $z_2$) is not aligned with the expected frame of reference ($x_1, y_1, z_1$), both in terms of orientation (or at least the orientation of the z axis) and position (or at least the height position), then the device may incorrectly determine a height metric corresponding to coordinate associated with a measured reflect point. This is because the coordinate is measured with respect to the nominal frame of reference, but the height metric is with respect to the actual environment (i.e. a frame of reference of the environment; also referred to herein as an expected frame of reference).

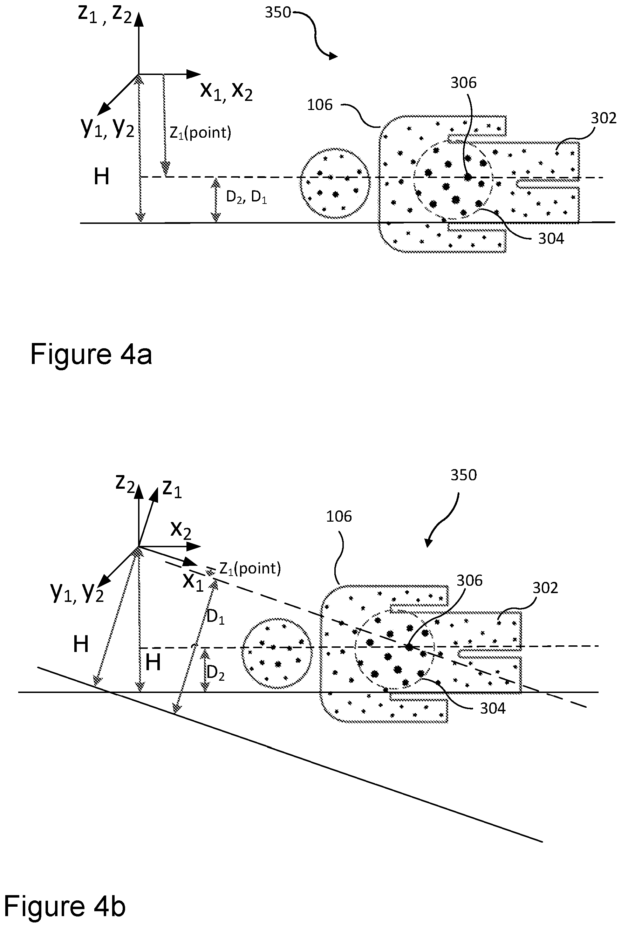

Figures 4a and 4b illustrate the issue with misaligned frames of reference in a height metric determination.

Figure 4a, similar to figure 3b, shows a height metric determination as described above, where the active reflected wave detector is correctly aligned with the frame of reference to the height metric determination i.e. the floor. Using the measured reflected wave data from the active reflected wave detector, the vertical displacement of a point from a reference position such as the floor, and thus a height of that point from the floor, can be determined accurately. In this case shown in figure 4a, the reference body has a point cloud from that represents a particular measured point 306, amongst other points. The measured the point 306 is measured as being (with respect to the z axis) as being at $z_1 = -z_1$(point). The height of the point 306 above the floor is calculated to be $D_1$, which equals $H - z_1$(point). In figure 4a, the device is mounted so that the active reflective wave detector's frame of reference of $(x_1, y_1, z_1)$ is aligned with the environment's frame of reference $(x_2, y_2, z_2)$. As a result, the calculated value of the height $D_1$ is equal to the actual height $D_2$.

Figure 4b illustrates a scenario where the active reflected wave detector is incorrectly aligned within the environment. Misalignment could be angular misalignments in any one or more of x, y or z axes, and/or may include a positional misalignment of the respective origins of one or more axes. However, in the illustrated example, misalignment is in only in the z-axis. When mounted on a wall, the active reflected wave detector may be misaligned with respect to the z axis through rotation about the x axis (which also causes a misalignment with the y axis), and/or rotation about the y axis (which also causes misalignment with the x axis). Figure 4b shows the latter case, where a rotation about the y axis has caused the x and z axes to be angularly misaligned such that the device is tilting forward away from the vertical wall defined by the $z_2$ axis.

Of course, misalignments may also occur in the x-y plane (where the device rotates about the z axis). However, misalignment by rotating in the z axis would not influence a vertical measurement (but simply misalign the useful field of view about the x-y axis). Therefore, such misalignments may be ignored in some embodiments.

In the case of the in figure 4b, the calculated height $D_1$ of the measured point 306 with respect to the active reflected wave detector's frame of reference is not equal to the actual height $D_2$, so is incorrect. If a determination of a condition or position of a person is taken based on a comparison of $D_1$, treated as the height metric, with a threshold value, this could cause a system implementing this device as a detector such as a fall detector to incorrectly determine that the person 302 is not in the fall position indicated in figure 4b, or in another fall position. In other scenarios a fall detector may determine that the person is in a fall position when they are not.

As will be appreciated, in addition to or instead of using the height $D_1$ of the point 306 as the height metric, the height metric used for the comparison with the threshold may be associated based on one or more points associated with the person 106. For example, the height metric could correspond to a spatial position of a weighted centre of the measurements (308 in figure 2) reflected from the person 106.

The embodiments disclosed herein facilitate or cause alignment of the 3D reflected wave measuring device (i.e. the active reflected wave detector 206) with a frame of reference of the environment. This is done using data from the orientation sensing device 210, which, in practice may be at least one of a gyroscope, a 3 axis accelerometer and a level sensor. In some embodiments the orientation sensing device 210 be 6-axis orientation sensing devices, the 6 axis consisting of 3 from a gyroscope and 3 from an accelerometer.

There are two main implementations of an alignment correction of the device in order that the device is correctly aligned to a frame of reference within the environment: 1) physical alignment; and 2) mathematical alignment. In either case, the purpose of the alignment correction is to re-align the orientation of the z-axis of the device with the z axis of the frame of reference in the environment, where misalignment has occurred because of a rotation of the device about the x and/or y axes. The alignment correction may also, or instead, be performed to re-align the x and/or y axes in the case where there has been a rotation of the device about the z axis. However, in some embodiments, misalignment in the x and/or y axes as a result of rotation of the device about the z axis is ignored.

The physical alignment is discussed first.

In this embodiment, and in its most fundamental configuration, the device utilises the orientation sensor 210 for the processor to provide feedback for a user to manually adjust a position of the device when installing the device.

Most simply the indication could be provided by one or more level meters (or representations of level meters). Two level meters respectively lying in perpendicular planes are preferable. As such, the user would adjust the pan and tilt of the device during installation in order to achieve the desired position as indicated by the level meters. The indication could in its most basic form be a binary output of level (to within a predefined accuracy) or not level.

In a related embodiment, the processor may give an indication when out of alignment of an extent and preferably also direction of the misalignment so that a person could easily align the device as needed. The indication may be provided by a visual and/or audio means on the device itself, or communicated to another device, for example a smartphone, tablet or computer.

In alternative embodiment where physical re-alignment occurs, the processor 202 may be coupled to drive actuators that control the position (pan and tilt) of the device. A closed loop control system may be implemented where the processor utilises the data from the orientation sensing device 210 in order to drive the actuators to move the device to minimise the misalignment between the device and the frame of reference (for example a horizontal plane lying at height $H_1$ above a point on a surface such as the floor, which in some embodiments is a horizontal surface).

The alignment of the device may be performed one or more times after installation of the device. The device may alert the user periodically if the position of the device has become misaligned with the intended frame of reference. With the motorised embodiment, the device may continuously monitor its orientation relative to the intended frame of reference.

Now discussed is the mathematical method of aligning the frame of reference of the orientation sensing device with the frame of reference of the environment.

In this embodiment, which is the preferred embodiment, the device is not physically reoriented, but rather, the angular difference (at least with respect to the z axis) between the second frame of reference and the first frame of reference (of the orientation sensing device) is compensated for so that the measurements made with respect to the first frame of reference are interpreted with respect to the second frame of reference.

The compensation can be achieved, for example using a rotation matrix. For example, the rotation matrix can be applied to either coordinates of respective measured wave reflections from the 3D reflected wave device, or to the first frame of reference.

Rotation matrices are well known in the in the operation of 3D coordinate systems.

Once the rotation matrix has been applied, the measurements made by the 3D reflected wave device are aligned to the intended frame of reference. Height metric measurements made based on the reflected wave measurements therefore provides an accurate determination of the height metric of an object, irrespective of the physical orientation of the device once installed.

The compensation may occur periodically, or before each determination of a height metric. It is also noted that the compensation, may be applied firstly on the raw data being received from the active reflective wave detector before any processing of the data to determine a weighted centre point is performed. Alternatively, the raw data may be processed to determine a weighted centre point (or other spatial location based on the raw data), and then compensation may be applied.

In either of the above implementations of the alignment, installation height data (H) may be stored in the memory of the device. The installation height data may comprise a height value at which the device is instructed to be installed on a wall. Alternatively installation height data may be data that is input by the installer of the device once the device has been installed. Furthermore, the installation height data may be data that is measured using the 3D reflected wave detector 206 (once correctly aligned with the intended frame of reference), for example based on a measured z-displacement from one or more identifiable reflection points on the floor, for example by a retroreflector placed on, or moving along, the floor.

As such, the vertical displacement of the 3D measurements with respect to the second frame of reference may be determined based on a vertical difference between the reflected data from the object and the installation height data.

Where it is referred herein to "aligning the frame of reference of the 3D reflected wave measuring device", this means aligning the frame of reference of the 3D reflected wave measuring device 206. It is the orientation sensing device 210 that provides the orientation data. However, the orientation sensing device package has a fixed and known positional relationship with respect to the 3D reflected wave measuring device, and so the orientation of the 3D reflected wave measuring device 206 may be determined with respect to the orientation sensing device 210 to that any differences between their respective orientations may be calibrated out (e.g. by mathematical alignment such as by using a rotation matrix, for example as described herein). Such a calibration may be performed in during manufacture of the device 102 for example, prior to its installation for use in the environment. It will be appreciated, however, that the aligning the frame of reference of the 3D reflected wave measuring device 206 to the frame of reference associated with the environment may be equivalently performed by aligning the orientation of the sensor sensing device 210 with the frame of reference of the environment, either physically or by mathematical manipulation, in conjunction with aligning the orientation of the sensor sensing device 210 with the frame of reference of the environment 3D reflected wave measuring device 206, either physically or by mathematical manipulation.

The phrase "A and/or B", "at least one of A and B" and "at least one of A or B" are each intended to include the following options, unless the contexts clearly requires otherwise: "A", "B", "at least A", "at least B", "at least one of A", "at least one of B", and all combinations thereof relating A with B, for example, "at least one of A and at least one of B",. Where used in a claim, the feature of the claim should be taken to be fulfilled if any one or more of those options is satisfied. If in a particular context any of those options are nonsensical or result in a duplicated option then, in that particular context, only the nonsensical option or duplicated option may be disregarded.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

## Claims

1. A fall detector device (102) for determining a height metric of an object (106) in an environment (100) using 3D coordinates associated with the object (106) in the environment for determining a fall condition of the object, comprising:

a 3D reflected wave measuring device (206) having a 3D coordinate system for measuring 3D coordinates of an object (106) in the environment for determining the height metric of the object in the environment, the 3D reflected wave measuring device (206) having a first frame of reference;

an orientation sensing device (210) to indicate an orientation of the 3D reflected wave measuring device (206) in an environment for aligning the first frame of reference with a second frame of reference that is associated with the environment (100);

a processor (202) coupled to the orientation sensing device (210) and configured to receive orientation data indicative of an orientation of the device (102), the processor being configured to align the first frame of reference with the second frame of reference by using the orientation data to compensate for an angular difference between the first frame of reference and the second frame of reference,

wherein the fall detector device (102) is mountable on a wall within the environment and the second frame of reference comprises a horizontal plane associated with the environment (100), and

wherein the processor (202) is configured to determine the height metric of the object (106) in the environment relative to the second frame of reference using 3D reflected wave measurements received by the 3D reflected wave measuring device (206).

2. A fall detector device according to claim 1, wherein the processor (202) is configured to compensate for the angular difference between the first frame of reference and the second frame of reference by applying a rotation matrix to one or more axes of the first frame of reference and/or to one or more coordinates of 3D reflected wave measurements received by the 3D reflected wave measuring device (206).

3. A fall detector device according to claim 1 or 2, wherein the processor (202) receives height data, the height data indicating a height of the device (102) in the environment, and wherein the processor (202) is configured to determine the height metric of the object (106) in the environment using the height data and the 3D reflected wave measurements received by the 3D reflected wave measuring device (206).

4. A fall detector device according any preceding claim, wherein the 3D reflected wave measuring device (206) is a radar device.

5. A fall detector device according to any preceding claim, wherein the orientation sensing device (210) comprises at least one of a gyroscope, a 3 axis accelerometer or a level sensor.

6. A fall detector device according to any preceding claim, wherein the height metric determined by the processor (202) comprises a height of a weighted centre of the 3D reflected wave measurements received by the 3D reflected wave measuring device (206), optionally wherein the height of the weighted centre of the 3D reflected wave measurements received by the 3D reflected wave measuring device (206) is based on a Radar Cross Section (RCS) of each 3D reflected wave measurement.

7. A fall detector device according to any one of claims 1 to 5, wherein the height metric determined by the processor (202) comprises a maximum height of the 3D reflected wave measurements received by the 3D reflected wave measuring device (206), or wherein the height metric determined by the processor (202) comprises an average height of the 3D reflected wave measurements received by the 3D reflected wave measuring device (206).

8. A method of using a fall detector device for determining a height metric of an object (106) in an environment (100) using 3D coordinates associated with the object (106) in the environment (100) for determining a fall condition of the object (106),

the device (102) comprising:

a 3D reflected wave measuring device (206) for measuring 3D coordinates of an object (106) in the environment (100) for determining the height metric of the object (106) in the environment, the 3D reflected wave measuring device (206) having a 3D coordinate system having a first frame of reference;

an orientation sensing device (210) to indicate an orientation of the 3D reflected wave measuring device (206) in an environment for aligning the first frame of reference with a second frame of reference that is associated with the environment;

a processor (202) coupled to the orientation sensing device and configured to

receive orientation data indicative of an orientation of the device (102), the method comprising: installing the device (102) in the environment by mounting the device on the wall, wherein the second frame of reference comprises a horizontal plane associated with the environment (100); aligning the first frame of reference with the second frame of reference using the orientation data to compensate for an angular difference between the first frame of reference and the second frame of reference; and determining the height metric of the object in the environment relative to the second frame of reference using 3D reflected wave measurements received by the 3D reflected wave measuring device (206).

9. A method according to claim 8, wherein the step of aligning is performed one or more times after installation of the fall detector device (102).

10. A method according to claim 8 or 9, wherein compensating for the angular difference between the first frame of reference and the second frame of reference comprises applying a rotation matrix to one or more axes of the first frame of reference and/or to one or more coordinates of 3D reflected wave measurements received by the 3D reflected wave measuring device (206).

11. A method according to any one of claims 8 to 10, wherein the installed height of the fall detector device (102) on the wall is stored in memory (208) in the device as height data, and the method comprises determining a height metric of the object in the environment relative to the second frame of reference using the height data and 3D reflected wave measurements received by the 3D reflected wave measuring device (206).

12. A method according to any one of claims 8 to 11, wherein the height metric comprises a height of a weighted centre of the 3D reflected wave measurements received by the 3D reflected wave measuring device (206), optionally wherein the height of the weighted centre of the 3D reflected wave measurements received by the 3D reflected wave measuring device (206) is based on a Radar Cross Section (RCS) of each 3D reflected wave measurement.

13. A method according to any one of claims 8 to 11, wherein the height metric comprises a maximum height of the 3D reflected wave measurements received by the 3D reflected wave measuring device (206), or wherein the height metric comprises an average height of the 3D reflected wave measurements received by the 3D reflected wave measuring device (206).

14. A method according to any one of claims 8 to 13, wherein the step of aligning the first frame of reference with the second frame of reference is performed at least one of one or more times after installation, or prior to each determination of the vertical displacement of an object (106) in the environment (100).

15. A method according any one of claims 8 to 14, wherein the 3D reflected wave measuring device (206) is a radar device, and/or wherein the orientation sensing device (210) comprises at least one of a gyroscope, a 3 axis accelerometer or a level sensor.

**Patentansprüche**

1. Sturzdetektorvorrichtung (102) zum Bestimmen einer Höhenmetrik eines Objekts (106) in einer Umgebung (100) unter Verwendung von 3D-Koordinaten, die mit dem Objekt (106) in der Umgebung assoziiert sind, zum Bestimmen eines Sturzzustandes des Objekts, umfassend:

eine 3D-Messvorrichtung (206) für reflektierte Wellen mit einem 3D-Koordinatensystem zum Messen von 3D-Koordinaten eines Objekts (106) in der Umgebung zum Bestimmen der Höhenmetrik des Objekts in der Umgebung, wobei die 3D-Messvorrichtung (206) für reflektierte Wellen ein erstes Bezugssystem aufweist; eine Orientierungserfassungsvorrichtung (210), um eine Orientierung der 3D-Messvorrichtung (206) für reflektierte Wellen in einer Umgebung anzugeben, und zwar zum Ausrichten des ersten Bezugssystems mit einem zweiten Bezugssystem, das mit der Umgebung (100) assoziiert ist; einen Prozessor (202), der mit der Orientierungserfassungsvorrichtung (210) gekoppelt und dafür konfiguriert ist, Orientierungsdaten zu empfangen, die eine Orientierung der Vorrichtung (102) kennzeichnen, wobei der Prozessor dafür konfiguriert ist, das erste Bezugssystem mit dem zweiten Bezugssystem auszurichten, indem er die Orientierungsdaten verwendet, um eine Winkeldifferenz zwischen dem ersten Bezugssystem und dem zweiten Bezugssystem auszugleichen, wobei die Sturzdetektorvorrichtung (102) an einer Wand innerhalb der Umgebung anbringbar ist und das zweite Bezugssystem eine horizontale Ebene umfasst, die mit der Umgebung (100) assoziiert ist, und wobei der Prozessor (202) dafür konfiguriert ist, die Höhenmetrik des Objekts (106) in der Umgebung relativ zu dem zweiten Bezugssystem unter Verwendung von 3D-Messungen reflektierter Wellen zu bestimmen, die durch die 3D-Messvorrichtung (206) für reflektierte Wellen empfangen werden.

2. Sturzdetektorvorrichtung nach Anspruch 1, wobei der Prozessor (202) dafür konfiguriert ist, die Winkeldifferenz zwischen dem ersten Bezugssystem und dem zweiten Bezugssystem durch Anwenden einer Rotationsmatrix auf eine oder mehrere Achsen des ersten Bezugssystems und/oder auf eine oder mehrere Koordinaten von 3D-Messungen reflektierter Wellen, die von der 3D-Messvorrichtung (206) für reflektierte Wellen empfangen werden, auszugleichen.

3. Sturzdetektorvorrichtung nach Anspruch 1 oder 2, wobei der Prozessor (202) Höhendaten empfängt, wobei die Höhendaten eine Höhe der Vorrichtung (102) in der Umgebung angeben, und wobei der Prozessor (202) dafür konfiguriert ist, die Höhenmetrik des Objekts (106) in der Umgebung unter Verwendung der Höhendaten und der von der 3D-Messvorrichtung (206) für reflektierte Wellen empfangenen 3D-Messungen reflektierter Wellen zu bestimmen.

4. Sturzdetektorvorrichtung nach einem der vorhergehenden Ansprüche, wobei die 3D-Messvorrichtung (206) für reflektierte Wellen eine Radarvorrichtung ist.

5. Sturzdetektorvorrichtung nach einem der vorhergehenden Ansprüche, wobei die Orientierungserfassungsvorrichtung (210) mindestens eines von Folgendem umfasst: ein Gyroskop, ein 3-Achsen-Beschleunigungssensor oder ein Niveausensor.

6. Sturzdetektorvorrichtung nach einem der vorhergehenden Ansprüche, wobei die durch den Prozessor (202) bestimmte Höhenmetrik eine Höhe eines gewichteten Zentrums der durch die 3D-Messvorrichtung (206) für reflektierte Wellen empfangenen 3D-Messungen reflektierter Wellen umfasst, wobei die Höhe des gewichteten Zentrums der durch die 3D-Messvorrichtung (206) für reflektierte Wellen empfangenen 3D-Messungen reflektierter Wellen optional auf einem Radarquerschnitt (RCS) jeder 3D-Messung reflektierter Wellen beruht.

7. Sturzdetektorvorrichtung nach einem der Ansprüche 1 bis 5, wobei die durch den Prozessor (202) bestimmte Höhenmetrik eine maximale Höhe der durch die 3D-Messvorrichtung (206) für reflektierte Wellen empfangenen 3D-Messungen reflektierter Wellen umfasst, oder wobei die durch den Prozessor (202) bestimmte Höhenmetrik eine durchschnittliche Höhe der durch die 3D-Messvorrichtung (206) für reflektierte Wellen empfangenen 3D-Messungen reflektierter Wellen umfasst.

8. Verfahren zum Verwenden einer Sturzdetektorvorrichtung zum Bestimmen einer Höhenmetrik eines Objekts (106) in einer Umgebung (100) unter Verwendung von 3D-Koordinaten, die mit dem Objekt (106) in der Umgebung (100) assoziiert sind, zum Bestimmen eines Sturzzustands des Objekts (106),

wobei die Vorrichtung (102) umfasst:

eine 3D-Messvorrichtung (206) für reflektierte Wellen zum Messen von 3D-Koordinaten eines Objekts (106) in der Umgebung (100) zum Bestimmen der Höhenmetrik des Objekts (106) in der Umgebung, wobei die 3D-Messvorrichtung (206) für reflektierte Wellen ein 3D-Koordinatensystem mit einem ersten Bezugssystem aufweist; eine Orientierungserfassungsvorrichtung (210), um eine Orientierung der 3D-Messvorrichtung (206) für reflektierte Wellen in einer Umgebung anzugeben, und zwar zum Ausrichten des ersten Bezugssystems mit einem zweiten Bezugssystem, das mit der Umgebung assoziiert ist; einen Prozessor (202), der mit der Orientierungserfassungsvorrichtung gekoppelt und dafür konfiguriert ist, Orientierungsdaten zu empfangen, die eine Orientierung der Vorrichtung (102) kennzeichnen, wobei das Verfahren umfasst:

Installieren der Vorrichtung (102) in der Umgebung durch Anbringen der Vorrichtung an der Wand, wobei das zweite Bezugssystem eine horizontale Ebene umfasst, die mit der Umgebung (100) assoziiert ist; Ausrichten des ersten Bezugssystems mit dem zweiten Bezugssystem unter Verwendung der Orientierungsdaten, um eine Winkeldifferenz zwischen dem ersten Bezugssystem und dem zweiten Bezugssystem auszugleichen; und Bestimmen der Höhenmetrik des Objekts in der Umgebung relativ zu dem zweiten Bezugssystem unter Verwendung von 3D-Messungen reflektierter Wellen, die durch die 3D-Messvorrichtung (206) für reflektierte Wellen empfangen werden.

9. Verfahren nach Anspruch 8, wobei der Schritt des Ausrichtens ein- oder mehrmals nach der Installation der Sturzdetektorvorrichtung (102) durchgeführt wird.

10. Verfahren nach Anspruch 8 oder 9, wobei das Ausgleichen der Winkeldifferenz zwischen dem ersten Bezugssystem und dem zweiten Bezugssystem umfasst: Anwenden einer Rotationsmatrix auf eine oder mehrere Achsen des ersten Bezugssystemes und/oder auf eine oder mehrere Koordinaten von 3D-Messungen reflektierter Wellen, die durch die 3D-Messvorrichtung (206) für reflektierte Wellen empfangen werden.

11. Verfahren nach einem der Ansprüche 8 bis 10, wobei die Installhöhe der Sturzdetektorvorrichtung (102) an der Wand in einem Speicher (208) in der Vorrichtung als Höhendaten gespeichert wird und das Verfahren umfasst: Bestimmen einer Höhenmetrik des Objekts in der Umgebung relativ zu dem zweiten Bezugssystem unter Verwendung der Höhendaten und der durch die 3D-Messvorrichtung (206) für reflektierte Wellen empfangenen 3D-Messungen reflektierter Wellen.

12. Verfahren nach einem der Ansprüche 8 bis 11, wobei die Höhenmetrik eine Höhe eines gewichteten Zentrums der durch die 3D-Messvorrichtung (206) für reflektierte Wellen empfangenen 3D-Messungen reflektierter Wellen umfasst, optional wobei die Höhe des gewichteten Zentrums der durch die 3D-Messvorrichtung (206) für reflektierte Wellen empfangenen 3D-Messungen reflektierter Wellen auf einem Radarquerschnitt (RCS) jeder 3D-Messung reflektierter Wellen beruht.

13. Verfahren nach einem der Ansprüche 8 bis 11, wobei die Höhenmetrik eine maximale Höhe der durch die 3D-Messvorrichtung (206) für reflektierte Wellen empfangenen 3D-Messungen reflektierter Wellen umfasst, oder wobei die Höhenmetrik eine durchschnittliche Höhe der durch die 3D-Messvorrichtung (206) für reflektierte Wellen empfangenen 3D-Messungen reflektierter Wellen umfasst.

14. Verfahren nach einem der Ansprüche 8 bis 13, wobei der Schritt des Ausrichtens des ersten Bezugssystems mit dem zweiten Bezugssystem mindestens einmal von einem oder mehreren Malen nach der Installation oder vor jeder Bestimmung der senkrechten Verschiebung eines Objekts (106) in der Umgebung (100) durchgeführt wird.

15. Verfahren nach einem der Ansprüche 8 bis 14, wobei die 3D-Messvorrichtung (206) für reflektierte Wellen eine Radarvorrichtung ist; und/oder wobei die Orientierungserfassungsvorrichtung (210) mindestens eines von Folgendem umfasst: ein Gyroskop, ein 3-Achsen-Beschleunigungssensor oder ein Niveausensor.

## Revendications

1. Dispositif détecteur de chute (102) pour déterminer une mesure de hauteur d'un objet (106) dans un environnement (100) en utilisant des coordonnées 3D associées à l'objet (106) dans l'environnement pour déterminer une condition de chute de l'objet, comprenant :

   un dispositif de mesure d'onde réfléchie 3D (206) comportant un système de coordonnées 3D pour mesurer des coordonnées 3D d'un objet (106) dans l'environnement pour déterminer la mesure de hauteur de l'objet dans l'environnement, le dispositif de mesure d'onde réfléchie 3D (206) disposant d'un premier système de référence ;
   un dispositif de détection d'orientation (210) pour indiquer une orientation du dispositif de mesure d'onde réfléchie 3D (206) dans un environnement pour aligner le premier système de référence avec un second système de référence qui est associé à l'environnement (100) ; et
   un processeur (202) couplé au dispositif de détection d'orientation (210) et configuré pour recevoir des données d'orientation indicatives d'une orientation du dispositif (102), le processeur étant configuré pour aligner le premier système de référence avec le second système de référence en utilisant les données d'orientation pour compenser une différence angulaire entre le premier système de référence et le système de référence,
   dans lequel le dispositif détecteur de chute (102) peut être monté sur un mur à l'intérieur de l'environnement et le second système de référence comprend un plan horizontal associé à l'environnement (100), et
   dans lequel le processeur (202) est configuré pour déterminer la mesure de hauteur de l'objet (106) dans l'environnement en relation avec le second système de référence en utilisant des mesures d'onde réfléchie 3D reçues par le dispositif de mesure d'onde réfléchie 3D (206).

2. Dispositif détecteur de chute selon la revendication 1, dans lequel le processeur (202) est configuré pour compenser la différence angulaire entre le premier système de référence et le second système de référence en appliquant une matrice de rotation à un ou plusieurs axes du premier système de référence et/ou à une ou plusieurs coordonnées de mesures d'onde réfléchie 3D reçues par le dispositif de mesure d'onde réfléchie 3D (206).

3. Dispositif détecteur de chute selon la revendication 1 ou 2, dans lequel le processeur (202) reçoit des données de hauteur, les données de hauteur indiquant une hauteur du dispositif (102) dans l'environnement, et dans lequel le processeur (202) est configuré pour déterminer la mesure de hauteur de l'objet (106) dans l'environnement en utilisant les données de hauteur et les mesures d'onde réfléchie 3D reçues par le dispositif de mesure d'onde réfléchie 3D (206).

4. Dispositif détecteur de chute selon l'une quelconque des revendications précédentes, dans lequel le dispositif de mesure d'onde réfléchie 3D (206) est un dispositif radar.

5. Dispositif détecteur de chute selon l'une quelconque des revendications précédentes, dans lequel le dispositif de détection d'orientation (210) comprend au moins un dispositif parmi un gyroscope, un accéléromètre à 3 axes ou un capteur de niveau.

6. Dispositif détecteur de chute selon l'une quelconque des revendications précédentes, dans lequel la mesure de hauteur déterminée par le processeur (202) comprend une hauteur d'un centre pondéré des mesures d'onde réfléchie 3D reçues par le dispositif de mesure d'onde réfléchie 3D (206), en option dans lequel la hauteur du centre pondéré des mesures d'onde réfléchie 3D reçues par le dispositif de mesure d'onde réfléchie 3D (206) est basée sur une surface équivalente radar (SER) de chaque mesure d'onde réfléchie 3D.

7. Dispositif détecteur de chute selon l'une quelconque des revendications 1 à 5, dans lequel la mesure de hauteur déterminée par le processeur (202) comprend une hauteur maximum des mesures d'onde réfléchie 3D reçues par le dispositif de mesure d'onde réfléchie 3D (206), ou dans lequel la mesure de hauteur déterminée par le processeur (202) comprend une hauteur moyenne des mesures d'onde réfléchie 3D reçues par le dispositif de mesure d'onde réfléchie 3D (206).

8. Procédé d'utilisation d'un dispositif détecteur de chute pour déterminer une mesure de hauteur d'un objet (106) dans un environnement (100) en utilisant des coordonnées 3D associées à l'objet (106) dans l'environnement (100) pour déterminer une condition de chute de l'objet (106),

le dispositif (102) comprenant :

un dispositif de mesure d'onde réfléchie 3D (206) pour mesurer des coordonnées 3D d'un objet (106) dans l'environnement (100) pour déterminer la mesure de hauteur de l'objet (106) dans l'environnement, le dispositif de mesure d'onde réfléchie 3D (206) comportant un système de coordonnées 3D disposant d'un premier système de référence ;

un dispositif de détection d'orientation (210) pour indiquer une orientation du dispositif de mesure d'onde réfléchie 3D (206) dans un environnement pour aligner le premier système de référence avec un second système de référence qui est associé à l'environnement ;

un processeur (202) couplé au dispositif de détection d'orientation et configuré pour recevoir des données d'orientation indicatives d'une orientation du dispositif (102),

le procédé comprenant :

l'installation du dispositif (102) dans l'environnement en montant le dispositif sur le mur, dans lequel le second système de référence comprend un plan horizontal associé à l'environnement (100) ;

l'alignement du premier système de référence avec le second système de référence en utilisant les données d'orientation pour compenser une différence angulaire entre le premier système de référence et le second système de référence ; et

la détermination de la mesure de hauteur de l'objet dans l'environnement en relation avec le second système de référence en utilisant des mesures d'onde réfléchie 3D reçues par le dispositif de mesure d'onde réfléchie 3D (206).

9. Procédé selon la revendication 8, dans lequel l'étape d'alignement est réalisée une ou plusieurs fois après l'installation du dispositif détecteur de chute (102).

10. Procédé selon la revendication 8 ou 9, dans lequel la compensation de la différence angulaire entre le premier système de référence et le second système de référence comprend l'application d'une matrice de rotation sur un ou plusieurs axes du premier système de référence et/ou sur une ou plusieurs coordonnées de mesures d'onde réfléchie 3D reçues par le dispositif de mesure d'onde réfléchie 3D (206).

11. Procédé selon l'une quelconque des revendications 8 à 10, dans lequel la hauteur d'installation du dispositif détecteur de chute (102) sur le mur est stockée dans une mémoire (208) dans le dispositif en tant que données de hauteur, et le procédé comprend la détermination d'une mesure de hauteur de l'objet dans l'environnement en relation avec le second système de référence en utilisant les données de hauteur et les mesures d'onde réfléchie 3D reçues par le dispositif de mesure d'onde réfléchie 3D (206).

**12.** Procédé selon l'une quelconque des revendications 8 à 11, dans lequel la mesure de hauteur comprend une hauteur d'un centre pondéré des mesures d'onde réfléchie 3D reçues par le dispositif de mesure d'onde réfléchie 3D (206), en option dans lequel la hauteur du centre pondéré des mesures d'onde réfléchie 3D reçues par le dispositif de mesure d'onde réfléchie 3D (206) est basée sur une surface équivalente radar (SER) de chaque mesure d'onde réfléchie 3D.

**13.** Procédé selon l'une quelconque des revendications 8 à 11, dans lequel la mesure de hauteur comprend une hauteur maximum des mesures d'onde réfléchie 3D reçues par le dispositif de mesure d'onde réfléchie 3D (206), ou dans lequel la mesure de hauteur comprend une hauteur moyenne des mesures d'onde réfléchie 3D reçues par le dispositif de mesure d'onde réfléchie 3D (206).

**14.** Procédé selon l'une quelconque des revendications 8 à 13, dans lequel l'étape d'alignement du premier système de référence avec le second système de référence est réalisée au moins une fois d'une ou de plusieurs fois après l'installation, ou avant chaque détermination du déplacement vertical d'un objet (106) dans l'environnement (100).

**15.** Procédé selon l'une quelconque des revendications 8 à 14, dans lequel le dispositif de mesure d'onde réfléchie 3D (206) est un dispositif de radar ; et/ou dans lequel le dispositif de détection d'orientation (210) comprend au moins un dispositif parmi un gyroscope, un accéléromètre à 3 axes ou un capteur de niveau.

Figure 1

Figure 2

Figure 3a

Figure 3b

Figure 4a

Figure 4b

**EP 4 153 047 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019195728 A1
- US 2016077202 A1
- US 2016040982 A1
- US 7916066 B1